# EUROPEAN PATENT APPLICATION

(11) **EP 2 266 563 A1**
(43) Date of publication of application: **29.12.2010**
(21) Application number: 09162499.9
(22) Date of filing: 11.06.2009
(51) Int. Cl.: A61K 31/485, A61P 43/00

(54) **Use of opioid receptor antagonists for acute treatment of paraphilic arousal states**

(71) Applicant: Charité-Universitätsmedizin Berlin (Charité), 10117 Berlin (DE)
(72) Inventor: Beier, Klaus, 14169 Berlin (DE); Dittgen, Michael, 99510 Apolda (DE); Dittgen, Elke, 99441 Kleinschwabhausen (DE); Pauls, Alfred, 13507 Berlin (DE)
(74) Representative: Gulde Hengelhaupt Ziebig & Schneider

(57) **Abstract**

The present invention is directed to an opioid receptor antagonist or a pharmaceutically acceptable salt thereof for use in a method of acute treatment of paraphilic sexual arousal states in patients suffering from paraphilia.

## Description

### BACKGROUND

Paraphilia refers to a condition characterized by powerful and persistent sexual interest other than in copulatory or precopulatory behaviour with phenotypically normal, consenting adult human partners.

Psychologists and psychiatrists codified paraphilias as disorders in the 1980 version of the *Diagnostic and Statistical Manual of Mental Disorders* (DSM-III). The current version of the DSM (DSM-IV-TR) describes paraphilias as conditions which "are characterized by recurrent, intense sexual urges, fantasies, or behaviours that involve unusual objects, activities, or situations and cause clinically significant distress or impairment in social, occupational, or other important areas of functioning," - thus fulfilling the criteria for disease. Paraphilias are similarly codified, defined and described in International Statistical Classification of Diseases and Related Health Problems (ICD-10), 10^{th} Revision, Version 2007, in chapter V, F65, issued by the WHO.

The exact criteria for a DSM-IV-TR diagnosis of paraphilia are:
"Paraphilias are defined by DSM-IV-TR as sexual disorders characterized by "recurrent, intense sexually arousing fantasies, sexual urges or behaviours generally involving (1) nonhuman objects, (2) the suffering or humiliation of oneself or one's partner, or (3) children or other nonconsenting persons that occur over a period of 6 months" (Criterion A), which "cause clinically significant distress or impairment in social, occupational, or other important areas of functioning" (Criterion B).

The treatment of paraphilias and related disorders has been challenging for patients and clinicians. Psychotherapy, self-help groups, and pharmacotherapy have all been used. Antiandrogenic drugs such as cyptroterone acetate and medroxyprogesterone have been widely used as therapy in men with paraphilia to reduce sex drive. However, their efficacy is limited and they have many unpleasant side effects, including breast growth, headaches, weight gain, and reduction in bone density. Even if compliance is good, only 60 to 80 percent of men benefit from this type of drug. Long-acting gonadotropin-releasing hormones, such as Triptorelin (Trelstar) which reduces the endogenous release of gonadotropin hormones, are also used. Psychostimulants have been used recently to augment the effects of serotonergic drugs in paraphiliacs. In theory, the prescription of a psychostimulant without pretreatment with a selective serotonin reuptake inhibitor or serotonin-specific reuptake inhibitor (SSRI) might further disinhibit sexual behaviour, but when taken together, the psychostimulant may actually reduce impulsive tendencies.

Recently the use of Naltrexon for long term treatment of obsessive sexual desire has been reported in two cases (Raymond et al., International Clinical Psychopharmacology, 2002; 201-205). These diseases are not grouped under paraphilias but form part of obsessive-compulsive disorders. In this study Naltrexon was administered regularly over a longer period and not in close connection with acute sexual excitement states. In the two cases a reduction in frequency of obsessive sexual desire could be observed after several treatments.

In another study (Ryback et al., Journal Clinical Psychiatry, 2004; 982-986) sentenced sexual offenders were treated with a regular daily dose of 100 to 200 mg Naltrexon. It is not reported that one of these sexual offenders indeed suffered from a paraphilia. Again Naltrexon was administered in a regular scheme over a longer period of time, independent from acute sexual excitement states.

Although in the two publications named above, an opioid receptor antagonist, namely Naltrexon, has been used for treatment of sexual disorders, opioid receptor antagonists have not been used or proposed for treatment of paraphilia and in particular not for acute treatment of paraphilic sexual arousal states.

One problem of the regular administration of drugs over a longer period of time is the occurrence of side effects that may be not tolerable and may effect the need for appropriate medication to counteract these side effects or to introduce intermitting periods of discontinuation of the original treatment schedule. Overall compliance and willingness to enter such a treatment are therefore reduced.

There remains a need for a medical treatment of paraphilia which avoids or alleviates one or more of the drawbacks of present treatment strategies. Thus, it is an objective of the present invention to provide such a medical treatment.

### DESCRIPTION OF THE INVENTION

The present invention relates to an opioid receptor antagonist or a pharmaceutically acceptable salt thereof for use in a method of acute treatment of paraphilic sexual arousal states in patients suffering from paraphilia.

The present invention is based on the surprising finding that administration of an opioid receptor antagonist to a patient suffering from paraphilia directly before, at the onset or during a paraphilic sexual arousal state is suitable to reduce the severity of or even to avoid or disrupt the development of such a paraphilic sexual arousal state. After treatment, the patient is able to control his behaviour and to avoid any actions that may harm the patient or his environment.

According to the present invention the patient is not administered with a medicament in a predetermined, regular long-term dosing scheme, but the patient is administered with said medicament only, if he is in acute need in order to acutely diminish a paraphilic sexual arousal state.

The use of the invention has the effect that paraphilic behaviour is controlled and/or suppressed efficiently while the patient is not exposed to regular doses of a drug, in particular of an opioid receptor antagonist or a pharmaceutically acceptable salt thereof. Thus, the likelihood of development of side effects is reduced and compliance and willingness to accept such treatment are increased.

The present invention encompasses an opioid receptor antagonist for use in a method of acute treatment of paraphilic sexual arousal states. As used herein, the term "treating" or "treatment" refers to reversing, alleviating or inhibiting the progress of a disease, disorder or condition, or one or more symptoms of such disease, disorder or condition, to which such term applies. As used herein, "treating" or "treatment" may also refer to decreasing the probability or incidence of the occurrence of a disease, disorder or condition in a patient as compared to the experience in an untreated control population, or as compared to the same patient prior to treatment. For example, as used herein, "treating" or "treatment" may refer to preventing a disease, disorder or condition, and may include delaying or preventing the onset of a disease, disorder or condition, or delaying or preventing the symptoms associated with a disease, disorder or condition. As used herein, "treating" or "treatment" may also refer to reducing the severity of a disease, disorder or condition or symptoms associated with such disease, disorder or condition prior to a patient's affliction with the disease, disorder or condition. Such prevention or reduction of the severity of a disease, disorder or condition prior to affliction relates to the administration of the opioid receptor antagonists of the present invention, as described herein, to a patient that is not at the time of administration afflicted with the disease, disorder or condition. As used herein "treating" or "treatment" may also refer to preventing the recurrence of a disease, disorder or condition or of one or more symptoms associated with such disease, disorder or condition. The terms "treatment" and "therapeutically," as used herein, refer to the act of treating, as "treating" is defined above.

The term "acute" as used herein defines that each treatment or administration is directly triggered by the presence or reasoned expectance of the onset of a particular paraphilic sexual arousal state. In particular acute treatment may be triggered by self-perception of signs of onset of a paraphilic sexual arousal state by the patient, by self-perception of an ongoing paraphilic sexual arousal state or before the patient is exposed to a situation, subject or subject matter suitable to elicit an acute paraphilic sexual arousal state in said patient. Preferably the term "acute" encompasses a time window starting 2 hours before the expected onset of a paraphilic sexual arousal state until any time point during the persistence of the paraphilic sexual arousal state of a particular patient. Even more preferred the term "acute" refers to a time windoe of not more than 1 hour before the expected onset of a paraphilic sexual arousal state, most preferably 30 min, 10 min or 5 min before the expected onset of a paraphilic sexual arousal state.

In the present invention an opioid receptor antagonist or a pharmaceutically acceptable salt thereof is used for acute treatment of patients suffering from paraphilia. Such patients are human subjects which are diagnosed by a medical practitioner or physician to suffer from a paraphilia in accordance with the criteria formulated either in DSM-IV-TR and/or ICD-10 who whish to maintain self-control. Preferably the patients suffers from one or more paraphilias selected from exhibitionism, fetishism, frotteurism, paedophilia, sexual masochism, sexual sadism, voyeurism, transvestic fetishism, telephone scatologia, necrophilia, partialism, zoophilia, coprophilia, klismaphilia, urophilia and/or emetophilia.

According to the invention an opioid receptor antagonist or a pharmaceutically acceptable salt thereof is used for acute treatment of a paraphilic sexual arousal state in a patient suffering from paraphilia. A paraphilic sexual arousal state is a sexual arousal state that is associated, caused or elicited by subject matter of the actual paraphilia the respective patient is suffering from. In case of paraphilic fetishism, the paraphilic sexual arousal state is a sexual arousal state of the patient caused by the respective fetish. Sexual arousal is the arousal of sexual desires in anticipation for sexual activity. Sexual arousal for a man is usually indicated by the swelling and erection of the penis. In a woman, sexual arousal usually leads to vaginal lubrication in anticipation of sexual intercourse.

Sexual arousal may cause different physical changes:

| **In females:** | **In males:** |
|---|---|
| • Erection of nipples | • Penile tumescence and erection |
| • Vaginal lubrication • Vasocongestion of the vaginal walls • Tumescence and erection of the clitoris and labia • Elevation of the cervix and uterus, and expansion of the back of the vagina | • The veins in the penis may become more prominent • Retraction and tightening of the foreskin, often exposing the glans penis if not normally exposed (unless the particular male is circumcised) • Emission of pre-ejaculatory fluid • Swelling of the testes |
| • Change in shape, color and size of the labia majora and labia minora | • Ascension of the testes • Tensing and thickening of the scrotum |

However, physical signs do not need to be a prerequisite for sexual activity.

In the present invention acute treatment of paraphilic sexual arousal states includes the treatment of paraphilic sexual arousal states at the onset of sexual arousal, e.g. in order to prevent, abate and/or control the development of the sexual arousal state, or during an acute paraphilic sexual arousal of the patient, e.g. in order to stop, shorten and/or alleviate the intensity or duration of an acute sexual paraphilic sexual arousal state. Acute treatment of a paraphilic sexual arousal state also comprises the administration of an opioid receptor antagonist or a pharmaceutically acceptable salt thereof to a patient suffering from paraphilia directly before the patient is exposed to a situation, subject or subject matter suitable to elicit an acute paraphilic sexual arousal state of said patient. The intention of such an acute administration is to prevent the occurrence of such a paraphilic sexual arousal state and/or to alleviate the sexual arousal state to an extent that the patient is able to positively control his behaviour for the duration of the acute sexual arousal state.

According to the present invention an opioid receptor antagonist or a pharmaceutically acceptable salt thereof, in particular an effective amount of an opioid receptor antagonist or a pharmaceutically acceptable salt thereof, is used for acute treatment of paraphilic sexual arousal states. According to the present invention one or more than one opioid receptor antagonist may be used for acute treatment of paraphilic sexual arousal states in a patient suffering from paraphilia.

Opioid receptor antagonists are compounds or mixtures of compounds that act on opioid receptors and prevent the body from responding to opiates and/or endorphins. Commonly used opioid receptor antagonists are competitive antagonists that bind to the opioid receptors with high affinity but do not activate the receptors. Thereby the opioid receptors are completely or partially blocked for binding of opiates and/or endorphins. Suitable opioid receptor antagonists are known to the person skilled in the art. Preferably the opioid receptor antagonist is a 4,5-epoxy-morphinan derivative. A 4,5-epoxy-morphinan derivative is a compound encompassing a substituted or unsubstituted morphinan backbone structure:

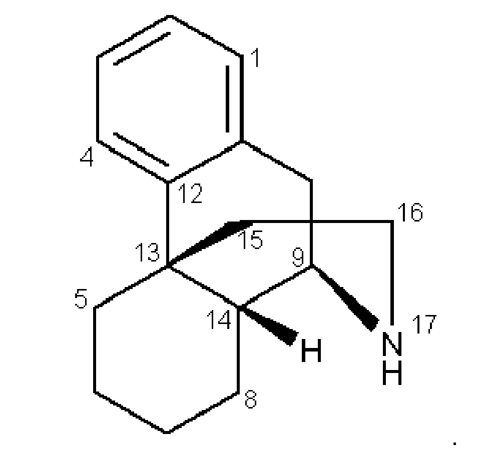

Particular preferred 4,5-epoxy-morphinan derivatives are 3,14-dihydroxy-4,5-epoxymorphinan-6-on compounds in which the hydrogen of N17 is substituted by a methyl, ethyl, propyl, butyl, isobutyl, cyclopropylmethyl, allyl, benzyl or phenethyl group. More preferably the opioid receptor antagonist is Naloxon (17-allyl-3,14-dihydroxy-4,5-epoxy-morphinan-6-on) and/or Naltrexon (17-cyclopropylmethyl-3,14-dihydroxy-4,5-epoxy-morphinan-6-on).

Pharmaceutically acceptable salts of the opioid receptor antagonist comprise:
- inorganic acid salts such as chlorides, hydrochlorides, sulfates, bisulfates, nitrates, hydrobromides, hydroiodides and phosphates;
- organic carboxylates such as acetates, lactates, citrates, oxalates, glutarates, malates, tartrates, bitartrates, fumarates, mandelates, maleates, succinates, benzoates and phthalates;
- organic sulfonates such as methanesulfonates, ethansulfonates, benzenesulfonates, p-toluenesulfonates and camphor-sulfonates.

According to the present invention, the opioid receptor antagonist is administered preferably at an effective dose. An "effective dose" is the dose of an opioid receptor antagonist that upon administration to a patient yields a measurable therapeutical effect with regard to the disease of interest. In the present invention an effective dose is the dose of an opioid receptor antagonist that upon administration to a patient yields a therapeutic effect with regard to paraphilic sexual arousal states in patients suffering from paraphilia. Preferably the opioid receptor antagonist is administered at a dose of not more than 5 mg/kg body weight per treatment or administration. In particular the opioid receptor antagonist can be administered at a dose of 0,1 µg/kg to 5000 µg/kg body weight per treatment or administration, preferably of 1 µg/kg to 2000 µg/kg body weight per treatment or administration. In order to prevent acute side effects to occur, it is recommended that the opioid receptor antagonist is administered at a maximum cumulative daily dose of not more than 10 mg/kg body weight.

When used in human therapy, the opioid receptor antagonists and/or their pharmaceutically acceptable salts will generally be administered as a formulation in association with one or more pharmaceutically acceptable excipients. The term "excipient" is used herein to describe any ingredient other than the opioid receptor antagonist or a pharmaceutically acceptable salt thereof. The choice of excipient or excipients will to a large extent depend on the particular mode of administration.

In a preferred embodiment the opioid receptor antagonist or a pharmaceutically acceptable salt thereof will be formulated together with an anionic macromolecule so that the anionic macromolecule will be present in the formulation in a concentration of not more than 0,5 Mol/kg, preferably in a concentration of 0,3 to 0,01 Mol/kg. Suitable anionic macromolecules may be water soluble sugars like glucose, saccharose or derivatives thereof; soluble starch or hydrolysates thereof; soluble cellulose or derivatives thereof; sorbitol, mannitol or sugar acids derived therefrom; polyacrylic acid or derivatives therefrom comprising one or more hydroxyl and or amino groups.

The opioid receptor antagonist or the pharmaceutically acceptable salt thereof may be administered orally. Oral administration may involve swallowing, so that the compound enters the gastrointestinal tract, or buccal or sublingual administration may be employed by which the compound enters the blood stream directly from the mouth.

Formulations suitable for oral administration include: solid formulations such as tablets; capsules containing particulates, liquids, or powders; lozenges (including liquid-filled); and chews; multi- and nanoparticles; gels; solid solutions; liposomes; films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The opioid receptor antagonists may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the opioid receptor antagonist or the pharmaceutically acceptable salt thereof may make up from 0,01 weight % to 80 weight % of the dosage form, more typically from 1 weight % to 75 weight % of the dosage form. In addition to the opioid receptor antagonist or the pharmaceutically acceptable salt thereof, tablets generally contain a disintegrant.

Examples of disintegrants include sodium starch glycolate, low-substituted sodium carboxymethyl cellulose, calcium carboxymethyl cellulose an derivatives thereof, croscarmellose sodium, crospovidone, polyvinylpyrrolidone, substituted methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% opioid receptor antagonist or the pharmaceutically acceptable salt thereof, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated. The formulation of tablets is exemplarily discussed in Pharmaceutical Dosage Forms: Tablets, Vol. 1, by H. Lieberman and L. Lachman (Marcel Dekker, New York, 1980).

Consumable oral films or wafers for human use are typically pliable water-soluble or water-swellable thin film dosage forms which may be rapidly dissolving or mucoadhesive and typically comprise an opioid receptor antagonist or the pharmaceutically acceptable salt thereof, a film-forming polymer, a binder, a solvent, a humectant, a plasticiser, a stabiliser or emulsifier, a viscosity-modifying agent and a solvent. Some components of the formulation may perform more than one function.

The opioid receptor antagonist or the pharmaceutically acceptable salt thereof may be water-soluble or insoluble. A water-soluble compound typically comprises from 0,01 weight % to 80 weight %, more typically from 1 weight % to 50 weight %, of the solutes. Less soluble compounds may comprise a greater proportion of the composition, typically up to 88 weight % of the solutes. Alternatively, the opioid receptor antagonist or the pharmaceutically acceptable salt thereof may be in the form of micro-sized or nano-sized beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films and/or wafers in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in U.S. Pat. No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The opioid receptor antagonist or the pharmaceutically acceptable salt thereof may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of the opioid receptor antagonist or the pharmaceutically acceptable salt thereof used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus the opioid receptor antagonist or the pharmaceutically acceptable salt thereof may be formulated as a solid, semisolid, or thixotropic liquid for administration as an implanted depot providing modified release of the active compound. Examples of such formulations include drug-coated stents and PGLApoly(dl-lactic-coglycolic)acid (PGLA) microspheres.

The opioid receptor antagonist or the pharmaceutically acceptable salt thereof may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated-see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (e.g. Powderject(TM), Bioject(TM), etc.) injection.

Formulations for topical administration may be formulated to be immediate and/or modified release. Modified release formulations include delayed-, sustained-, pulsed-, controlled-, targeted and programmed release.

The opioid receptor antagonist or the pharmaceutically acceptable salt thereof can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the opioid receptor antagonist or the pharmaceutically acceptable salt thereof comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the opioid receptor antagonist or the pharmaceutically acceptable salt thereof product may be micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the opioid receptor antagonist or the pharmaceutically acceptable salt thereof, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 µg to 20 mg of the opioid receptor antagonist or the pharmaceutically acceptable salt thereof per actuation and the actuation volume may vary from 1 µl to 100 µl. A typical formulation may comprise an opioid receptor antagonist or the pharmaceutically acceptable salt thereof, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly glycol Poly (lactide-co-glycolide)(PLGA), amine-modified polyvinyl alcohol (PVA) backbone and amine-PVA-g-PLGA modified release formulations.

In the case of dry powder inhalers and aerosols, the dosage unit is determined by means of a valve which delivers a metered amount. Units in accordance with the invention are typically arranged to administer a metered dose or "puff" containing from 0.001 mg to 10 mg of the opioid receptor antagonist or the pharmaceutically acceptable salt thereof.

The opioid receptor antagonist or the pharmaceutically acceptable salt thereof are particularly suitable for an administration by inhalation.

The opioid receptor antagonist or the pharmaceutically acceptable salt thereof may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The opioid receptor antagonist or the pharmaceutically acceptable salt thereof may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The opioid receptor antagonist or the pharmaceutically acceptable salt thereof may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the opioid receptor antagonist or the pharmaceutically acceptable salt thereof, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Preferably the opioid receptor antagonist or the pharmaceutically acceptable salt thereof is administered as a formulation for immediate release of the opioid receptor antagonist. Thus, the opioid receptor antagonist is readily available and able to provide its therapeutic activity to the patient in need. One advantage of the use of an immediate release formulation is that the formulation may when administered immediately at the onset of a paraphilic sexual arousal state can efficiently allow the patient to control his behaviour.

In particular the opioid receptor antagonist or a pharmaceutically acceptable salt thereof may preferably be administered orally, transdermally, nasally and/or mucosally.

The opioid receptor antagonist or a pharmaceutically acceptable salt thereof may preferably be administered in form of a tablet, a capsule, a liquid, a powder, a lozenge, a wafer, a transmucosal therapeutic system, a nasal spray and/or an aerosol.

The present invention also refers to:
Opioid receptor antagonist or a pharmaceutically acceptable salt thereof for use in a method of acute treatment of paraphilic sexual arousal states in patients suffering from paraphilia.
Opioid receptor antagonist or a pharmaceutically acceptable salt thereof of claim 1, wherein the opioid receptor antagonist is a 4,5-epoxy-morphinan derivative.
Opioid receptor antagonist or a pharmaceutically acceptable salt thereof of anyone of the preceding claims, wherein the opioid receptor antagonist is Naloxon and/or Naltrexon.
Opioid receptor antagonist or a pharmaceutically acceptable salt thereof of anyone of the preceding claims, wherein the opioid receptor antagonist or a pharmaceutically acceptable salt thereof is administered at a dose of not more than 5 mg/kg body weight per administration cycle.
Opioid receptor antagonist or a pharmaceutically acceptable salt thereof of anyone of the preceding claims, wherein the opioid receptor antogonist or a pharmaceutically acceptable salt thereof is administered at a dose of 0,1 µg/kg to 5000 µg/kg body weight per administration cycle, preferably of 1 µg/kg to 2000 µg/kg body weight per administration cycle.
Opioid receptor antagonist or a pharmaceutically acceptable salt thereof of anyone of the preceding claims, wherein the opioid receptor antagonist or a pharmaceutically acceptable salt thereof is administered at a cumulative daily dose of not more than 10 mg/kg body weight.
Opioid receptor antagonist or a pharmaceutically acceptable salt thereof of anyone of the preceding claims, wherein the opioid receptor antagonist or a pharmaceutically acceptable salt thereof is administered in a formulation for immediate release of the opioid receptor antagonist.
Opioid receptor antagonist or a pharmaceutically acceptable salt thereof of anyone of the preceding claims, wherein the opioid receptor antagonist or a pharmaceutically acceptable salt thereof is administered in a formulation comprising 0,5 Mol/kg of an anionic macromolecule.
Opioid receptor antagonist or a pharmaceutically acceptable salt thereof of anyone of the preceding claims, wherein the opioid receptor antagonist or a pharmaceutically acceptable salt thereof is administered orally, transdermally, nasally and/or mucosally.
Opioid receptor antagonist or a pharmaceutically acceptable salt thereof of anyone of the preceding claims, wherein the opioid receptor antagonist or a pharmaceutically acceptable salt thereof is administered in form of a tablet, a capsule, a liquid, a powder, a lozenge, a wafer, a transmucosal therapeutic system, a nasal spray and/or an aerosol.
Opioid receptor antagonist or a pharmaceutically acceptable salt thereof of anyone of the preceding claims, wherein the opioid receptor antagonist or a pharmaceutically acceptable salt thereof is administered to a patient suffering from paraphilia at the onset of or during an acute paraphilic sexual arousal state.
Opioid receptor antagonist or a pharmaceutically acceptable salt thereof of anyone of the preceding claims, wherein the opioid receptor antagonist or a pharmaceutically acceptable salt thereof is administered to a patient suffering from paraphilia directly before the patient is exposed to a situation, subject or subject matter suitable to elicit a paraphilic sexual arousal state of said patient.
Opioid receptor antagonist or a pharmaceutically acceptable salt thereof of anyone of the preceding claims, wherein the receptor antagonist or a pharmaceutically acceptable salt thereof is administered to a patient suffering from exhibitionism, fetishism, frotteurism, paedophilia, sexual masochism, sexual sadism, voyeurism, transvestic fetishism, telephone scatalogia, necrophilia, partialism, zoophilia, coprophilia, klismaphilia, urophilia and/or emetophilia.
Opioid receptor antagonist or a pharmaceutically acceptable salt thereof of anyone of the preceding claims, wherein more than one opioid receptor antagonist or a pharmaceutically acceptable salt thereof is used for acute treatment of paraphilic sexual arousal states.

A method of acute treatment of paraphilic sexual arousal states in patients suffering from paraphilia, wherein an effective amount of an opioid receptor antagonist of the invention or a pharmaceutically acceptable salt thereof is used and/or administered to a patient in need thereof.

Use of an opioid receptor antagonist of the invention or a pharmaceutically acceptable salt thereof for acute treatment of paraphilic sexual arousal states in patients suffering from paraphilia.

Use of an opioid receptor antagonist of the invention or a pharmaceutically acceptable salt thereof for the manufacture of a medicament for the acute treatment of paraphilic sexual arousal states in patients suffering from paraphilia.

In the following the present invention is explained in more detail by way of examples.

### EXAMPLES

### EXAMPLE 1 Formulations

### Lozenge:

One tablet comprises

| | |
|---|---|
| Naloxonhydrochloride | 2mg |
| Peppermint aroma | 0,1 mg |
| Sorbitol | 1350mg |
| Saccharin/sodium | 0,5mg |
| Macrogol 6000 | 0,6mg |
| Talcum | 0,4mg |
| Magnesium stearat | 0,1 mg |

The lozenge is prepared by known methods.

### Wafer for oral administration:

One wafer comprises

| | |
|---|---|
| Naloxonhydrochloride | 1mg |
| Hydroxypropylmethylcellulose | 95mg |

Method for manufacturing of 10 wafers:
10mg Naloxonhydrochloride is dissolved to completion in 70ml ethanol/H₂O (1:1). 950mg Hydroxypropylmethylcellulose (HPMC) is added and dissolved to completion. The resulting solution is degassed, streaked out and dried. The resulting thin, transparent film has a thickness of 50 to 100µm. The film is cut into appropriate Wafer portions.

### Bioadhesive tablet for buccal administration:

One tablet comprises

| | |
|---|---|
| Naloxonhydrochloride | 1,5mg |

And other ingredients like carboxyvinylpolymere, lactose, starch, sodiumcarboxymethylstarch and magnesium stearat.
The tablet is prepared by known methods for manufacturing of bioadhesive tablets for buccal administration.

### Nasal spray:

One puff comprises

| | |
|---|---|
| Naloxonhydrochloride | 0,2mg. |

Other ingredients are benzyl alcohol, sorbitol, acetic acid, sodiumchloride, sodiumacetate and H₂O.
The nasal spray is prepared by known methods for manufacturing of nasal sprays.

### Aerosol for oral administration (pump spray):

One puff comprises

| | |
|---|---|
| Naloxonhydrochloride | 0,15mg. |

Other ingredients are non-polar solvents (e.g. H₂O) 30% and polar solvents (e.g. glycole or alcohol) 70%.
The aerosol and pump spray is prepared by known methods for manufacturing of aerosols and pump sprays.

### EXAMPLE 2 Case reports

In the clinical application (two patients with paraphilic patterns of sexual excitation/arousal, both paedophiles, but with individual symptomatology) the following observations were made:
1. Observations in a patient with marked expression of symptoms:
   The patient is a 45 years old man with paedophile inclination towards sexually immature girls (in addition to his predominant orientation towards adult women). In his first marriage he had repeatedly abused the daughter of his wife between the ages of 7 and 10 years (touching the genitals of the girl, as well as having himself touched until reaching the climax). These crimes have not yet become known or led to any form of legal prosecution, and this first marriage is divorced since several years. Owing to his job as music teacher the patient was increasingly in contact with girls of the preferred age during the recent two years; the patient was very worried (and this worry was justified) that he might exploit the situation for the sexual abuse of his pupils. In the course of treatment at the Institute for Sexual Medicine of the Charité University Clinic of Berlin the patient has changed his professional field so that he is mainly involved in the treatment of grown-ups. For the few contacts with female pupils of the preferred age he regularly uses one or two puffs of nasal spray containing naloxone (specification of nasal spray: one puff comprises 0,2mg Naloxonhydrochloride; other ingredients are benzyl alcohol, sorbitol, acetic acid, sodiumchloride, sodiumacetate and H₂O; the nasal spray is prepared by known methods for manufacturing of nasal sprays). He notices a sufficient reduction of his sexual interest in the child, so that he can concentrate on the pedagogical aspects of music relevant in the lesson. This leads to a marked improvement in the feeling of control over the situation. On a scale reaching from 0 (no control of impulse) to 10 (very good control of impulse) the patient placed himself between 2 and 3 before the application of naloxone. Three to five minutes after the application he rated his impulse control between 7 and 8.
2. Observations in a patient with moderate expression of symptoms:
   The patient is a 43 years old man suffering enormously from a predominant hebephilic inclination towards boys between the ages of 12 and 14 years (in hebephilia only the peripubertal stages of development lead to sexual arousal, neither the body of children nor that of adults). Owing to his activities as a football trainer in exactly this age bracket he was frequently exposed to stimulating situations, particularly since he was very popular with the boys and their parents. In the course of his treatment at the Institute for Sexual Medicine of the Charité University Clinic of Berlin he gave up training the boys but now works in the administration of the club as a volunteer. He still has contact with the boys, and has fallen in love with one of them (the mother is single and the boy spontaneously seeks contact with the patient). Up until now the patient has not committed any sexual crimes, but he is afraid that the impulse might break through in socially uncontrolled situations. In the therapy he learned to avoid such situations; with respect to foreseeable contacts with the boy in the context of the activities of the club he decided to take one or two puffs of nasally applied naloxone (specification of nasal spray: one puff comprises 0,2mg Naloxonhydrochloride; other ingredients are benzyl alcohol, sorbitol, acetic acid, sodiumchloride, sodiumacetate and H₂O; the nasal spray is prepared by known methods for manufacturing of nasal sprays). He notices a reduction of his sexual interest in the boy, leading to reassuring awareness of control over the situation. On a scale reaching from 0 (no control of impulse) to 10 (very good control of impulse) the patient placed himself between 3 and 4 before the application of naloxone. Two to four minutes after the application he rated his impulse control between 8 and 9.

## Claims

1. Opioid receptor antagonist or a pharmaceutically acceptable salt thereof for acute treatment of paraphilic sexual arousal states in patients suffering from paraphilia.

2. Opioid receptor antagonist or a pharmaceutically acceptable salt thereof of claim 1, wherein the opioid receptor antagonist is a 4,5-epoxy-morphinan derivative.

3. Opioid receptor antagonist or a pharmaceutically acceptable salt thereof of anyone of the preceding claims, wherein the opioid receptor antagonist is Naloxon and/or Naltrexon.

4. Opioid receptor antagonist or a pharmaceutically acceptable salt thereof of anyone of the preceding claims, wherein the opioid receptor antagonist or a pharmaceutically acceptable salt thereof is administered at a dose of not more than 5 mg/kg body weight per administration cycle.

5. Opioid receptor antagonist or a pharmaceutically acceptable salt thereof of anyone of the preceding claims, wherein the opioid receptor antogonist or a pharmaceutically acceptable salt thereof is administered at a dose of 0,1 µg/kg to 5000 µg/kg body weight per administration cycle, preferably of 1 µg/kg to 2000 µg/kg body weight per administration cycle.

6. Opioid receptor antagonist or a pharmaceutically acceptable salt thereof of anyone of the preceding claims, wherein the opioid receptor antagonist or a pharmaceutically acceptable salt thereof is administered at a cumulative daily dose of not more than 10 mg/kg body weight.

7. Opioid receptor antagonist or a pharmaceutically acceptable salt thereof of anyone of the preceding claims, wherein the opioid receptor antagonist or a pharmaceutically acceptable salt thereof is administered in a formulation for immediate release of the opioid receptor antagonist.

8. Opioid receptor antagonist or a pharmaceutically acceptable salt thereof of anyone of the preceding claims, wherein the opioid receptor antagonist or a pharmaceutically acceptable salt thereof is administered in a formulation comprising 0,5 Mol/kg of an anionic macromolecule.

9. Opioid receptor antagonist or a pharmaceutically acceptable salt thereof of anyone of the preceding claims, wherein the opioid receptor antagonist or a pharmaceutically acceptable salt thereof is administered orally, transdermally, nasally and/or mucosally.

10. Opioid receptor antagonist or a pharmaceutically acceptable salt thereof of anyone of the preceding claims, wherein the opioid receptor antagonist or a pharmaceutically acceptable salt thereof is administered in form of a tablet, a capsule, a liquid, a powder, a lozenge, a wafer, a transmucosal therapeutic system, a nasal spray and/or an aerosol.

11. Opioid receptor antagonist or a pharmaceutically acceptable salt thereof of anyone of the preceding claims, wherein the opioid receptor antagonist or a pharmaceutically acceptable salt thereof is administered to a patient suffering from paraphilia at the onset of or during an acute paraphilic sexual arousal state.

12. Opioid receptor antagonist or a pharmaceutically acceptable salt thereof of anyone of the preceding claims, wherein the opioid receptor antagonist or a pharmaceutically acceptable salt thereof is administered to a patient suffering from paraphilia directly before the patient is exposed to a situation, subject or subject matter suitable to elicit a paraphilic sexual arousal state of said patient.

13. Opioid receptor antagonist or a pharmaceutically acceptable salt thereof of anyone of the preceding claims, wherein the receptor antagonist or a pharmaceutically acceptable salt thereof is administered to a patient suffering from exhibitionism, fetishism, frotteurism, paedophilia, sexual masochism, sexual sadism, voyeurism, transvestic fetishism, telephone scatalogia, necrophilia, partialism, zoophilia, coprophilia, klismaphilia, urophilia and/or emetophilia.

14. Opioid receptor antagonist or a pharmaceutically acceptable salt thereof of anyone of the preceding claims, wherein more than one opioid receptor antagonist or a pharmaceutically acceptable salt thereof is used for acute treatment of paraphilic sexual arousal states.

15. Use of an opioid receptor antagonist or a pharmaceutically acceptable salt thereof of one of the preceding claims for the manufacture of a medicament for the acute treatment of paraphilic sexual arousal states in patients suffering from paraphilia.
